# EUROPÄISCHE PATENTSCHRIFT

(11) **EP 3 017 064 B1**
(45) Veröffentlichungstag und Bekanntmachung des Hinweises auf die Patenterteilung: **31.05.2017**
(21) Anmeldenummer: 14739093.4
(22) Anmeldetag: 02.07.2014
(51) Int. Cl.: C12Q 1/68

(54) **VERFAHREN UND ANORDNUNG ZUR ERFASSUNG VON BINDUNGSEREIGNISSEN VON MOLEKÜLEN**
METHOD AND ARRANGEMENT FOR RECORDING MOLECULAR BINDING PHENOMENA
PROCÉDÉ ET DISPOSITIF POUR DÉTECTER DES ÉVÉNEMENTS DE LIAISON DE MOLÉCULES

(30) Priorität: 02.07.2013 DE 102013011304
(43) Veröffentlichungstag der Anmeldung: 11.05.2016
(73) Patentinhaber: Technische Universität Dresden, 01069 Dresden (DE)
(72) Erfinder: LIN, Weilin, 01307 Dresden (DE); WIEDUWILD, Robert, 01069 Dresden (DE); MANNOCCI, Luca, 5074 Eiken (CH); HERRMANN, Jana, 01324 Dresden (DE); ZHANG, Yixin, 01219 Dresden (DE); REDDAVIDE, Francesco, 01099 Dresden (DE)
(74) Vertreter: Pfenning, Meinig & Partner mbB
(86) Internationale Anmeldenummer: PCT/EP2014/064097
(87) Internationale Veröffentlichungsnummer: WO 2015/000978

(56) Entgegenhaltungen:
- WO-A1-95/16055
- WO-A1-03/059929
- WO-A2-01/79548
- WO-A2-2008/036802
- WO-A2-2012/085069
- US-A1- 2010 029 502
- NICOLE C HAUSER ET AL: "Utilising the left-helical conformation of L-DNA for analysing different marker types on a single universal microarray platform", NUCLEIC ACIDS RESEARCH, OXFORD UNIVERSITY PRESS, GB, Bd. 34, Nr. 18, 1. Oktober 2006 (2006-10-01), Seiten 5101-5111, XP002667723, ISSN: 1362-4962, DOI: 10.1093/NAR/GKL671
- WANG Y ET AL: "MICROARRAYS ASSEMBLED IN MICROFLUIDIC CHIPS FABRICATED FROM POLY(METHYL METHACRYLATE) FOR THE DETECTION OF LOW-ABUNDANT DNA MUTATIONS", ANALYTICAL CHEMISTRY, AMERICAN CHEMICAL SOCIETY, US, Bd. 75, Nr. 5, 1. März 2003 (2003-03-01), Seiten 1130-1140, XP001170273, ISSN: 0003-2700, DOI: 10.1021/AC020683W
- BRANDSTETTER T ET AL: "A polymer-based DNA biochip platform for human papilloma virus genotyping", JOURNAL OF VIROLOGICAL METHODS, ELSEVIER BV, NL, Bd. 163, Nr. 1, 1. Januar 2010 (2010-01-01), Seiten 40-48, XP026788543, ISSN: 0166-0934, DOI: 10.1016/J.JVIROMET.2009.07.027 [gefunden am 2009-08-05]
- ABRAMOV M ET AL: "HNA and ANA high-affinity arrays for detections of DNA and RNA single-base mismatches", BIOSENSORS AND BIOELECTRONICS, ELSEVIER BV, NL, Bd. 23, Nr. 11, 15. Juni 2008 (2008-06-15) , Seiten 1728-1732, XP022625063, ISSN: 0956-5663, DOI: 10.1016/J.BIOS.2008.01.033 [gefunden am 2008-02-13]
- YEW CHUNG TANG ET AL.: "DNA-directed assembly of protein microarrays", FRONTIERS IN BIOSCIENCE, Bd. 13, 1. Mai 2008 (2008-05-01), Seiten 5755-5771, XP9180539,

## Beschreibung

Die Erfindung betrifft ein Verfahren und eine Anordnung zur Erfassung von Bindungsereignissen von Molekülen, die insbesondere für Hochdurchsatzanwendungen geeignet sind.

Das Kennen und Verstehen von Bindungsereignissen und Wechselwirkungen zwischen Biomolekülen stellt ein wesentliches Arbeitsfeld der biologischen, pharmazeutischen und medizinischen Forschung dar. Ziel ist es dabei, mögliche Bindungspartner aufzuspüren und deren Signalwege zu charakterisieren, um mit den daraus gewonnenen Erkenntnissen Therapien oder Medikamente entwickeln zu können. Entdeckt werden pharmazeutisch relevante Wirkstoffe beispielsweise mithilfe von Screening-Verfahren, bei denen eine Vielzahl von in Bibliotheken katalogisierter Moleküle oder Strukturen gegeneinander oder gegen bekannte Zielstrukturen auf ihre Bindungseigenschaften getestet werden. Somit können aus der großen Anzahl katalogisierter Strukturen beispielsweise diejenigen Strukturen ermittelt werden, die an ein krankheiterregendes Protein binden und es dadurch hemmen. Zur Erfassung solcher Strukturen werden die bekannten zu untersuchenden Zielstrukturen (Targets) für gewöhnlich als stationäre Phase auf der Oberfläche eines Biochips kovalent gebunden, wobei die so immobilisierten Targets dann durch Überströmen mit einer mobilen Phase, einer Flüssigkeit in der eine Vielzahl möglicher Bindungsstrukturen (Moleküle bzw. Molekülteile) enthalten sind, in Kontakt gebracht werden. Während diesem Kontakt kann beispielsweise auf Oberflächenplasmonresonanz (SPR) oder auf quartzcrystall-microbalance (QCM) basierenden Verfahren die Assoziation und Dissoziation zwischen den immobilisierten Targets und den mobilen Strukturen (Moleküle bzw. Molekülteile) bestimmt werden.

Es liegt auf der Hand, dass es angesichts der Vielzahl möglicher Bindungspartner erforderlich ist, geeignete Hochdurchsatz-Verfahren anzuwenden, mit denen die Vielzahl möglicher Bindungskombinationen in einer vertretbaren Zeit bewältigt werden können. Insbesondere durch die stetige Erweiterung der Substanzbibliotheken steigt der Druck, höhere Durchsatzraten zu erreichen. Aufgrund der substrat- bzw. biochipseitigen kovalenten Anbindung der Targets ist es allerdings nur unter extremen Bedingungen möglich, ein Ablösen der so immobilisierten Targets zu erreichen. Es ist daher erforderlich, einen Biochip für jedes zu untersuchende Target erneut aufwendig zu präparieren. Insbesondere bei der Durchführung von Screenings mit mehreren Targets im Hochdurchsatz eignen sich derartige Biochips daher nicht in ausreichendem Maß.

Es sind bereits Verfahren bekannt, bei denen die Bindung zwischen Biotin und Streptavidin für eine Regeneration eines Biochips ausgenutzt wird. Dabei wird Streptavidin kovalent an der Oberfläche des Biochips gebunden. In einem weiteren Schritt binden biotinylierte Liganden an dem immobilisierten Streptavidin und bilden eine stationäre Phase. In der stationären Phase immobilisiert, können die Liganden auf ihre Bindungseigenschaften getestet werden. Aufgrund der hohen Bindungsaffinität von Biotin zu Streptavidin, die der Bindungsstärke einer kovalenten chemischen Bindung gleichwertig ist, sind jedoch extreme Bedingungen (bspw. hohe Temperaturen) notwendig, um eine Dissoziation der biotinylierten Liganden von dem kovalent gebundenen Streptavidin zu bewirken. Obwohl eine Ablösung der mit den biotinylierten Liganden gebildete stationäre Phase prinzipiell möglich ist, führen die extremen Bedingungen zu einer teilweise oder vollständigen Denaturierung des Streptavidinmoleküls, so dass eine weitere Immobilisierung mit biotinylierten Liganden an diesen Strepatvidinmolekülen nicht oder nur mit verringerter Bindungsstärke möglich ist.

Aufgabe der vorliegenden Erfindung ist es daher, Möglichkeiten für eine reversible Immobilisierung von Liganden anzugeben.

Erfindungsgemäß wird diese Aufgabe mit einem Verfahren gemäß Anspruch 1 und einer Anordnung nach Anspruch 9 gelöst.

Vorteilhafte Ausgestaltungen der vorliegenden Erfindung können den untergeordneten Ansprüchen entnommen werden.

Das erfindungsgemäße Verfahren umfasst die Merkmale gemäß Anspruch 1. Bei dem erfindungsgemäßen Verfahren zur Erfassung von Bindungsereignissen von Molekülen an einer bioaktiven Oberfläche wird in einem Schritt (I) an mindestens ein erstes Molekül, das durch eine kovalente Bindung an einer bioaktiven Oberfläche immobilisiert ist, mindestens ein zweites Molekül unter Bildung mindestens einer spezifischen nicht-kovalenten Bindung gebunden.

Das zweite Molekül kann derart funktionalisiert sein, dass es zur Anbindung/Kopplung weiterer dritter oder vierter Moleküle fähig ist. Durch Kontaktieren mit einer Zielstruktur (d.h. einem Analyten) enthaltenden mobilen Phase [Schritt (Ii)] können dann kinetische Experimente (Assoziationen und Dissoziationen) zwischen den immobilisierten zweiten, dritten oder vierten Molekülen und den mobilen Zielstrukturen (Analyten) durchgeführt werden.

In einem weiteren Schritt (II) wird die mindestens eine spezifische nicht-kovalente Bindung durch Zuführung einer Pufferlösung wieder gelöst. Dabei kann unter dem Lösen der spezifischen nicht-kovalenten Bindung eine Dissoziation des zweiten Moleküls von dem ersten Molekül verstanden werden, wobei das kovalent gebundene erste Molekül an der bioaktiven Oberfläche verbleibt und das zweite Molekül einschließlich aller daran gebundenen Moleküle von der Oberfläche entfernt werden. Im Anschluss an Schritt (II) sind die Schritte (I) und (II) mit den selben zweiten und/oder dritten funktionalsierten Molekülen und Analyten oder anderen zweiten und/oder dritten funktionalsierten Molekülen und Analyten mindestens einmal wieder-holbar.

Unter der bioaktiven Oberfläche soll eine Oberfläche verstanden werden, die aufgrund ihrer Materialeigenschaften geeignet ist und/oder derart funktionalisiert bzw. durch chemische Modifikation funktionalisierbar ist, dass an ihr zumindest das erste Molekül immobilisiert werden kann.

Die bioaktive Oberfläche kann daher auch auf unterschiedlichen geometrischen Formen ausgebildet sein. So kann die bioaktive Oberfläche beispielsweise auf dafür vorgesehene Biosensorchips oder in Bio-Assays ausgebildet sein oder eine sphärische Gestalt haben, wenn sie auf Nano-Partikeln oder Beads ausgebildet ist. Die bioaktive Oberfläche kann also auf der Oberfläche verschiedenartiger geometrischer Gebilde ausgebildet sein.

Während der Schritte (I) und (II) kann die bioaktive Oberfläche stets mit einer Pufferlösung (Laufpuffer) bedeckt sein. Im Schritt (I) kann ein Bindungspuffer verwendet werden, der ein besonderes für zu bindende Moleküle bindungsfreundliches Milieu aufweist.

Zum Lösen der zwischen dem mindestens einen ersten und dem mindestens einen zweiten Molekül gebildeten spezifischen nicht-kovalenten Bindung, kann die bioaktive Oberfläche im Schritt (II) mit einer Pufferlösung kontaktiert werden, die mindestens ein zum Lösen der spezifischen nicht-kovalenten Bindung geeignetes Molekül aufweist, das spezifisch an das zweite Molekül bindet und eine Dissoziationskonstante K_{D}(I) zu dem zweiten Molekül aufweist, die kleiner ist als eine Dissoziationskonstante K_{D}(II) des mindestens einen ersten Moleküls zu dem mindestens einen zweiten Molekül.

Dadurch, dass das zum Lösen zugeführte Molekül eine Dissoziationskonstante K_{D}(I) zu dem zweiten Molekül aufweist, die kleiner ist als eine Dissoziationskonstante K_{D}(II) des mindestens einen ersten Moleküls zu dem mindestens einen zweiten Molekül, ist das zugeführte Molekül auch zum Lösen der spezifischen nicht-kovalenten Bindung geeignet, da das zum Lösen zugeführte Molekül eine höhere Bindungsaffinität zu dem zweiten Molekül aufweist als das erste Molekül zu dem zweiten Molekül, so dass das erste Molekül von dem zweiten Molekül verdrängt wird. Besonders schonend kann das Lösen erfolgen, wenn die Konzentration des zum Ablösen geeigneten Moleküls während des Kontaktierens mit der Pufferlösung sukzessive oder kontinuierlich erhöht wird. Dabei sollte zumindest eine Konzentration des zum Ablösen geeigneten Moleküls erreicht werden, die der Konzentration des ersten auf der bioaktiven Oberfläche immobilisierten Moleküls entspricht.

Alternativ kann erfindungsgemäß ein Lösen der spezifischen nicht-kovalenten Bindung auch dadurch erreicht werden, dass die bioaktive Oberfläche im Schritt (II) mit einer Pufferlösung kontaktiert wird, die einen pH-Wert von pH 1 bis 4, bevorzugt kleiner als pH 3 aufweist. Ein schonendes Ablösen kann hierbei durch eine langsame Absenkung des pH-Wertes erreicht werden, wobei ein pH Wert von 1 nicht unterschritten werden sollte.

Die spezifische nicht-kovalente Anbindung des mindestens einen zweiten Moleküls an das mindestens eine erste Molekül kann als Immobilisierung einer aus dem mindestens einen zweiten Molekül gebildeten stationären Phase betrachtet werden, die im Schritt (II) auf einfache Weise durch eine Änderung des Puffersystems abgelöst werden kann, während eine untere Phase, die aus dem mindestens einen ersten Molekül gebildet ist, aufgrund der kovalenten Anbindung auf der bioaktiven Oberfläche verbleibt und das zweite Molekül einschließlich aller daran gebundenen Moleküle von der Oberfläche entfernt werden. Die mit dem mindestens einen zweiten Molekül gebildete stationäre Phase kann daher als temporäre stationäre Phase angesehen werden, wobei aufeinanderfolgende temporäre stationäre Phasen aufgrund unterschiedlicher Immobilisierungen/Funktionalisierungen unterschiedliche Eigenschaften aufweisen können.

Als untere Phase kann also die Phase verstanden werden, die mit dem mindestens einen ersten Molekül gebildet wird und dazu geeignet ist, die mit dem mindestens einen zweiten Molekül gebildete stationäre Phase chemisch zu binden. Es können aber auch komplexartig aufgebaute, an der bioaktiven Oberfläche kovalent gebundene erste Moleküle als untere Phase verwendet werden. Dies können bspw. an aktivierte Carboxylgruppen gekoppelte Diaminolinker sein, die das erste Molekül kovalent binden, wobei das erste Molekül eine entsprechende Amino-Funktionalisierung aufweisen sollte. Es ist außerdem denkbar, dass das erste Molekül in einer festen oder gelartigen Matrix auf der bioaktiven Oberfläche, bspw. eines Biosensorchips immobilisiert wird.

Nach Ablösen der mit dem mindestens einen zweiten Molekül gebildeten stationären Phase, kann im Anschluss an Schritt (II) wieder eine Anbindung einer weiteren mit mindestens einem zweiten Molekül gebildeten stationären Phase erfolgen.

Vorteilhafterweise sollte die bioaktive Oberfläche im Schritt (II) mit einem vorgebbaren Volumenstrom der Pufferlösung überströmt werden, um abgelöste zweite Moleküle und daran gebundener weiterer Moleküle gezielt abführen zu können. Das Verfahren eignet sich daher insbesondere für Anwendungen in Durchflusszellen, in denen eine Änderung von Fluidströmen auf einfache Weise realisiert werden kann.

Das erfindungsgemäße Verfahren eignet sich insbesondere auch für Hochdurchsatz-Anwendungen, da eine Vielzahl aufeinanderfolgender Beladungen und Ablösungen verschiedener zweiter Moleküle, d.h. verschiedener temporärer stationärer Phasen ohne wesentliche Unterbrechungen durchgeführt werden können.

Zusätzlich zu der mit dem zweiten Molekül gebildeten stationären Phase können in Schritt (I) auch weitere Moleküle zur Immobilisierung zugeführt werden. So kann in Schritt (I) auch eine Anbindung des mindestens einen dritten, optional des mindestens einen vierten Moleküls, insbesondere über eine kooperative Bindung, an das mindestens eine zweite Molekül erfolgen. So können stationäre Phasen mit unterschiedlichen Eigenschaften erhalten werden.

Vorzugsweise sollte dabei ein erstes Molekül eingesetzt werden, das eine Dissoziationskonstante K_{D}(II) zu dem mindestens einen zweiten Molekül aufweist die größer ist als die Dissoziationskonstante K_{D}(III) von dem mindestens einen zweiten Molekül zu dem mindestens einen dritten oder vierten Molekül. Dadurch kann gewährleistet werden, dass auch eine aus mehreren dritten und vierten Molekülen gebildete stationäre Phase in Schritt (II) sicher von der mit dem mindestens einen ersten Molekül gebildeten unteren Phase abgelöst werden kann, ohne dass die zweiten, dritten und/oder vierten Moleküle während des Eluierens, d.h. während des Lösens der nicht-kovalenten spezifischen Bindung (zwischen dem ersten und dem zweiten Molekül) in Schritt (II) dissoziieren. So können aus den zweiten, dritten und vierten Molekülen während des erstens Schrittes (I) gebildete Komplexe im Ganzen von der unteren Phase abgelöst und abgeführt werden.

Es kann auch ein erstes Molekül verwendet werden, das eine erste einzelsträngige DNA (desoxyribonucleic acid)/RNA (ribonucleic acid) oder Moleküle wie z.B. PNA (peptide nucleic acid) oder LNA (locked nucleic acid) enthält oder daraus besteht, an der mindestens ein zweites Molekül, das eine zweite einzelsträngige DNA/ RNA oder Moleküle PNA oder LNA enthält oder daraus besteht, zumindest bereichsweise komplementär gebunden wird. Dabei wird die stationäre Phase von den zweiten DNA/ RNA Einzelsträngen oder den Molekülen PNA oder LNA gebildet, die zumindest bereichswiese unter Bildung von DNA/ RNA Doppelsträngen oder PNA bzw. LNA Molekülkomplexen an die immobilisierten ersten DNA, RNA, PNA oder LNA hybridisieren bzw. binden. In diesem Fall sollte eine physiologische Pufferlösung mit einem pH-Wert von pH 6 bis pH 8 eingesetzt werden.

Für den Fall, dass als erstes Molekül eine erste einzelsträngige DNA oder RNA und als zweites Molekül eine zweite einzelsträngige DNA oder RNA verwendet wird, wobei die zweiten einzelsträngigen DNA oder RNA als stationäre Phase zumindest bereichsweise komplementär an die immobilisierte kovalent gebundenen ersten DNA oder RNA hybridisieren, sollte die bioaktive Oberfläche im Schritt (II) mit einer Pufferlösung kontaktiert werden, die einen pH-Wert kleiner als 3 aufweist, um das Lösen der stationären Phase zu bewirken. Bei der Verwendung von PNA oder LNA als erste und zweite Moleküle kann dabei gleichermaßen vorgegangen werden.

Infolge der pH-Wert-Änderung bei der Kontaktierung mit der Pufferlösung, denaturieren die Moleküle DNA/RNA, wobei die im Schritt (I) gebildeten DNA/RNA Doppelstränge zu DNA/RNA Einzelsträngen dissoziieren. Die dissoziierten Bestandteile, d.h. insbesondere die zweiten DNA/RNA Einzelstränge können dann beispielsweise mit der Strömung der Pufferlösung abgeführt werden. Zur Regenerierung und wiederholten Anbindung an die immobilisierten DNA/RNA Einzelstränge kann ein Neutralisationspuffers (z.B. Phosphatpuffer pH 7,4) zugeführt werden, wodurch im Bereich der immobilisierten ersten Moleküle ein pH-Wert erreicht werden kann, der dem Ausgangs-pH-Wert entspricht.

Das eingesetzte dritte Molekül kann einen Liganden aufweisen oder daraus bestehen, wobei der Ligand bevorzugt kovalent an dem dritten Molekül gebunden wird oder gebunden ist. Insbesondere enthält das dritte Molekül einen (sog. zweiten) Ligand, der chemisch kovalent an das dritte Molekül gebunden ist. Besonders bevorzugt ist der erste und/oder zweite Ligand auf der gesamten bioaktiven Oberfläche verteilt d.h. die bioaktive Oberfläche ist homogen mit dem ersten und/oder zweiten Ligand belegt. Der erste und zweite Ligand können verschieden oder identisch sein. Der Ligand und/oder zweite Ligand kann aus einer Gruppe bestehend aus Proteinen, Peptiden, Lipiden, DNA, RNA, PNA, LNA, Oligosacchariden und niedermolekularen Verbindungen mit einer Molekülmasse von ≤ 20.000 Da, bevorzugt ≤ 10.000 Da, besonders bevorzugt ≤ 3000 Da, insbesondere ≤ 900 Da, sowie Kombinationen davon, ausgewählt sein. Insbesondere bei der Verwendung von DNA/RNA als erstens und zweites Molekül, kann der Ligand beispielsweise in Form einer Gensonde (Nukleotid-Sequenz) an die immobilisierte DNA/RNA hybridisieren. Der Ligand kann aber auch in Form eines Proteins an der DNA/RNA gekoppelt sein.

Wie vorstehend bereits beschrieben, können, insbesondere zwischen den Schritten (I) und (II), weitere Schritte (Ii), beispielsweise zum Erfassen von Ligand-Analyt Bindungsereignissen, bevorzugt mittels Oberflächenplasmonresonanz-Spektroskopie (SPR), Lichtinterferenzmessung (LIM) und/oder Quarzkristall-Mikrowaagen (QCM), durchgeführt werden, ohne dass dabei die Bindungsstabilität der an der unteren Phase gebundenen stationären Phase beeinflusst wird. Bevorzugt wird die bioaktive Oberfläche mit einer Lösung enthaltend mindestens ein bestimmtes, bevorzugt nur ein bestimmtes, Analytmolekül (z.B. ein Protein als Analytmolekül) kontaktiert. Anschließend wird durch eine analytische Messung das Bindungsereignis von dem Analytmolekül an die bioaktive Oberfläche bestimmt. Als Bindungspartner für das Molekül aus der Lösung kommt hierbei der Ligand in Betracht, der an das mindestens eine zweite Molekül gebunden ist. Enthält das dritte Molekül ebenfalls einen (sog. zweiten) Liganden, so kommt als Bindungspartner auch dieser Ligand in Betracht, wobei die Bindung besonders stark ist, wenn beide Liganden an der Bindung zu dem Analytmolekül beteiligt sind. Das erste, zweite und/oder dritte Molekül dienen lediglich als Plattform für den/die Liganden und sollen an der Bindung zum Analyten nicht beteiligt sein. So können mit dem erfindungsgemäßen Verfahren gezielt und auf reversible Art und Weise potentielle Liganden für einen bekannten Analyten angeboten werden und Bindungsereignisse zwischen Analytmolekülen und Liganden gemessen werden. Insbesondere bei der Anwendung mit und auf Partikeln oder Beads können auf ALPHA (Amplified Luminescent Proximity Homogeneous Assay) basierenden Methoden (PerkinElmer) zur Erfassung von Protein-Protein Interaktion eingesetzt werden. Denkbar ist außerdem der Einsatz von fluoreszenzbasierten Partikel-Sortiersystemen, wie z.B. Durchflusszytometrie bzw. FACS (Fluorescence activated cell sorting), bei denen Molekülanbindungen bzw. Molekül-Molekül Interaktionen durch Fluoreszenz/Chemolumineszenz detektiert werden können. In diesem Fall können weitere Schritte/Inkubationen, beispielswiese zur Markierung mit Fluoreszenzfarbstoffen notwendig sein.

Zur Ausbildung der unteren Phase, kann die auf Nano-Partikeln, Beads, Bioassays oder Biosensorchips ausgebildete bioaktive Oberfläche aktivierbare Carboxylgruppen aufweisen, an die weitere (erste) Moleküle ankoppelbar sind.

Die erfindungsgemäße Anordnung enthält die Merkmale gemäß Anspruch 9. Die erfindungsgemäße Anordnung zur Erfassung von Bindungsereignissen von Molekülen weist mindestens ein erstes Molekül auf, das an einer bioaktiven Oberfläche durch eine kovalente Bindung immobilisiert ist. Die erfindungsgemäße Anordnung weist weiterhin mindestens ein zweites Molekül auf, das durch eine spezifische nicht-kovalente Bindung an dem mindestens einen ersten Molekül gebunden ist, wobei die spezifische nicht-kovalente Bindung lösbar ist.

Der entscheidende Vorteil der erfindungsgemäßen Anordnung besteht darin, dass eine mit dem zweiten Molekül gebildete temporäre stationäre Phase immobilisierbar ist, die nach Bedarf wieder entfernt werden kann, so dass die bioaktive Oberfläche für eine Anbindung weiterer zweiter Moleküle zur Verfügung steht. Vorteilhafterweise wird die aus dem mindestens einen ersten Molekül gebildete untere Phase durch das Ablösen der temporären stationären Phase nicht in ihren Bindungseigenschaften beeinflusst und kann daher nahezu vollständig wiederhergestellt werden, ohne dass eine aufwendige Präparation erforderlich ist.

Wie vorstehend bereits angeführt, wird unter der bioaktiven Oberfläche eine Oberfläche verstanden, die aufgrund ihrer Materialeigenschaften geeignet ist und/oder derart funktionalisiert bzw. durch chemische Modifikation funktionalisierbar ist, dass an ihr zumindest das erste Molekül kovalent immobilisierbar ist. Die erfindungsgemäße Anordnung eignet sich daher insbesondere für Biosensorchips, Bioassays, Nano-Partikel, Beads und Bead-Schüttungen (bzw. Formkörperschüttungen) und insbesondere für Durchflusszellen.

Die erfindungsgemäße Anordnung bzw. zumindest die bioaktive Oberfläche sollte mit einer physiologischen Pufferlösung (pH Neutral) benetzt sein, die vor Austrocknung schützt. Hierzu kann die erfindungsgemäße Anordnung auch von einem Gehäuse umgeben sein.

An dem mindestens einen zweiten Molekül kann vorzugsweise mindestens ein drittes und/oder viertes Molekül (wobei es sich bei dem vierten Molekül um das Analytmolekül handelt), insbesondere über eine kooperative Bindung, gebunden sein. Dadurch kann die stationäre Phase eine Vielzahl von Ausgestaltungsformen, beispielswiese eine Immobilisierung miteinander wechselwirkender Paare von Liganden und Analyten, aufweisen.

Das Lösen der zwischen dem mindestens einen ersten Molekül und dem mindestens einen zweiten Molekül gebildeten spezifischen nicht kovalenten Bindung kann bei Kontaktierung mit einer Pufferlösung erreicht werden, die mindestens ein zum Lösen der spezifischen nicht-kovalenten Bindung geeignetes Molekül enthält, das eine Dissoziationskonstante K_{D}(I) zu dem zweiten Molekül aufweist, die kleiner ist als eine Dissoziationskonstante K_{D}(II) des mindestens einen ersten Moleküls zu dem mindestens einen zweiten Molekül.

Das erste Molekül kann eine Dissoziationskonstante K_{D}(II) zu dem mindestens einen zweiten Molekül aufweisen, die größer ist als die Dissoziationskonstante K_{D}(III) von dem mindestens einen zweiten Molekül zu dem mindestens einen dritten oder vierten Molekül. Dadurch kann eine Ablösbarkeit einer mit dem mindestens einen zweiten und dem dritten und/oder vierten Molekül gebildeten stationären Phase gewährleistet werden, ohne dass die stationäre Phase selbst dissoziiert. Aus dem mindestens einen zweiten und dem dritten und/oder vierten Molekül gebildete Komplexe bleiben somit auch nach dem Ablösen der stationären Phase erhalten und werden somit von der Oberfläche entfernt.

In einer weiteren vorteilhaften Ausführungsform der erfindungsgemäßen Anordnung kann das erste Molekül auch mindestens eine erste einzelsträngige DNA/RNA bzw. PNA oder LNA enthalten oder daraus gebildet sein, an der ein eine zweite einzelsträngige DNA/RNA bzw. PNA oder LNA enthaltendes oder daraus bestehendes zweites Molekül zumindest bereichsweise komplementär gebunden ist. In diesem Fall bildet die zweite DNA/RNA bzw. PNA oder LNA die ablösbare stationäre Phase, wobei die zwischen der ersten DNA/RNA bzw. PNA oder LNA und der zweiten DNA/RNA bzw. PNA oder LNA gebildeten spezifischen nicht-kovalenten Bindungen bei Kontaktierung mit einer Pufferlösung lösbar sind, die einen pH-Wert aufweist der kleiner ist als pH 3.

Zur Bestimmung von Bindungsereignissen zwischen Liganden und Analyten (Zielstrukturen), kann die stationäre Phase mit einer mobilen flüssigen Phase überströmt oder kontaktiert sein, in der die Analyten vorhanden sind. Neben dem mindestens einen zweiten Molekül kann das mindestens eine dritte Molekül einen Liganden aufweisen oder daraus bestehen, wobei der Ligand bevorzugt kovalent an dem dritten Molekül gebunden ist. Besonders bevorzugt ist der erste und/oder zweite Ligand auf der gesamten bioaktiven Oberfläche verteilt d.h. die bioaktive Oberfläche ist homogen mit dem ersten und/oder zweiten Ligand belegt. Bei den Liganden kann es sich beispielsweise um Proteine, Peptide, Lipide, DNA PNA, LNA und/oder RNA, Oligosaccharide, niedermolekulare Verbindungen (small molecules) mit einer Molekülmasse von ≤ 20.000 Da, bevorzugt ≤ 10.000 Da, besonders bevorzugt ≤ 3000 Da, insbesondere ≤ 900 Da, sowie Kombinationen davon, handeln. Während des Überströmens/Kontaktierens mit der mobilen Phase können die darin enthaltenden Analyten (Zielstrukturen) mit den immobilisierten Liganden wechselwirken.

In einer weiteren Ausführungsform kann die untere Phase der erfindungsgemäßen Anordnung weitere kovalent gebundene, zur Immobilisierung des ersten Moleküls geeignete Moleküle oder aus Molekülen gebildete Komplexe aufweisen. Bei diesen Molekülen kann es sich um Moleküle handeln, die eine Carboxylgruppe aufweisen.

Die erfindungsgemäße Anordnung eignet sich generell zur analytischen Erfassung von Bindungsereignissen von Molekülen (z.B. von auf der Anordnung immobilisierten Liganden und bestimmten Analytmolekülen), insbesondere für Hochdurchsatz-Anwendungen in Durchflusszellen und für Messungen mittels Oberflächen-Plasmon-Resonanz (SPR), Lichtinterferenzmessung (LIM) und/oder über Quarzkristallmikrowaage (QCM). Es können mit der Anordnung selekiv Bindungsereignisse von bestimmten Analytmolekülen aus einer Lösung an die bioaktive Oberfläche der Anordnung erfasst werden. Als Bindungspartner für das Analytmolekül aus der Lösung kommt hierbei der Ligand in Betracht, den das mindestens eine zweite Molekül enthält. Weist das dritte Mokekül ebenfalls einen Liganden auf, so kommt als Bindungspartner auch dieser Ligand in Betracht, wobei die Bindung besonders stark ist, wenn beide Liganden an der Bindung zu dem Analytmolekül beteiligt sind. Das erste, zweite und/oder dritte Molekül dienen lediglich als Plattform für den/die Liganden und sollen an der Bindung zum Analyten nicht beteiligt sein. So können mit der erfindungsgemäßen Anordnung gezielt und auf reversible Art und Weise potentielle Liganden für einen bekannten Analyten angeboten werden und Bindungsereignisse zwischen Analytmolekülen und Liganden gemessen werden. Zur Erfassung von Molekül-Molekül Interaktionen kann die erfindungsgemäße Anordnung, insbesondere bei der Anwendung mit oder an Partikeln oder Beads, auch für Messverfahren/Nachweisverfahren eingesetzt werden, die auf dem Prinzip der fluoreszenzaktivierten Partikelsortierung, wie z.B. FACS (Fluorescence activated cell sorting) Durchflusszytometrie basieren. Es versteht sich, dass in diesem Fall geeignete Fluoreszenzmarker (Markierungen) zum Einsatz kommen, die spezifisch an mindestens ein auf der bioaktiven Oberfläche gebundenes oder immobilisiertes Molekül binden.

Eine weitere Anwendung findet die erfindungsgemäße Anordnung in Verbindung mit auf ALPHA.

Nachfolgend werden die vorliegende Erfindung und ihre vorteilhaften Ausgestaltungen anhand von Ausführungsbeispielen beschrieben.

Es zeigt:
- Figur 1:: eine Beladung und Regenerierung eines Biosensorchips,
- Figur 2a: eine Beispiel einer reversible Hybridisierung von DNA-Einzelsträngen (A';A) anhand eines QCM-Signal-Diagramms
- Figuren 2b-2c: Beispielexperimente zur Erfassung von Bindungsereignissen (Assoziation und Dissoziation) zwischen immobilisierten und mobilen Molekülen anhand von QCM-Signal-Diagrammen
- Figur 3: eine schematische Darstellung des erfindungsgemäßen Verfahrensprinzips anhand eines Ausführungsbeispiels

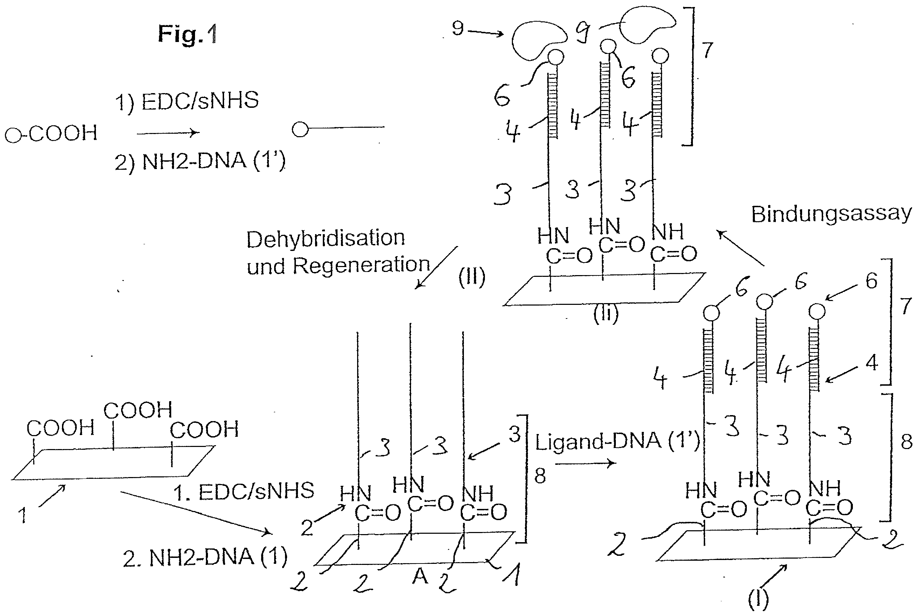

Die Figur 1 zeigt eine Beladung und Regenerierung eines Biosensorchips, bei dem der Biosensorchip im Ausgangszustand A eine Immobilisierung kovalent gebundener DNA Einzelstränge 3 als erstes Molekül aufweist. Dabei sind die DNA Einzelstränge 3 auf der bioaktiven Oberfläche 1 an kovalent gebundene Komplexe 2 kovalent immobilisiert. Mit dem Bezugszeichen 8 ist eine untere Phase gekennzeichnet, die zumindest aus den Komplexen 2 mit den daran kovalent gebundenen DNA Einzelsträngen 3 gebildet ist. In Schritt (I) des Verfahrens wird die untere Phase 8 mit einer Pufferlösung kontaktiert, in der zweite DNA Einzelstränge 4 als zweites Molekül enthalten sind. Dabei weisen die zweiten DNA Einzelstränge 4 kovalent gekoppelte Liganden 6 auf. Während der Kontaktierung hybridisieren die zweiten DNA Einzelstränge 4 komplementär, d.h. spezifisch nicht-kovalent an die immobilisierten DNA Einzelstränge 3 unter Bildung von DNA Doppelsträngen. Mit dem Bezugszeichen 7 ist dabei der Bereich dargestellt, der die obere stationäre Phase bildet.

Im Verfahrensschritt (Ii) wird die stationäre Phase 7 durch Überströmen mit einer mobilen Phase kontaktiert, wobei in der mobilen Phase vorhandene Strukturen 9 (Analyten) an den Liganden 6 assoziieren können. Während des Verfahrensschritts (Ii) kann die Assoziation und Dissoziation beispielsweise mittels Quarzkristall-Mikrowaagen ermittelt werden.

Zur Regeneration (Ablösen der temporären stationären Phase, Bezugszeichen 7) wird die bioaktive Oberfläche 1 in Schritt (II) mit einer Pufferlösung durch Überströmen mit einer vorgebbaren Strömungsgeschwindigkeit kontaktiert, wobei die Pufferlösung anfänglich einen pH Wert aufweist, der im Bereich von pH 8 bis pH 6 liegt und während des Kontaktierens bis zum Erreichen eines pH-Werts kleiner als 3 langsam verringert wird.

Infolge der pH-Wert Änderung während der Kontaktierung mit der Pufferlösung denaturieren die aus den DNA Einzelsträngen 3 und 4 gebildeten Doppelstränge, wobei die zweiten DNA Einzelstränge 4 von den ersten immobilisierten DNA Einzelsträngen 3 dissoziieren. Dabei wird die stationäre Phase 7 gelöst und mit der Strömung der Pufferlösung abgeführt. Abschließend wird ein Neutralisationspuffer oder Laufpuffer zugeführt, der einen physiologischen pH-Wert im Bereich von pH 6 bis pH 7 aufweist, wobei die immobilisierten DNA Einzelstränge 3 renaturieren können.

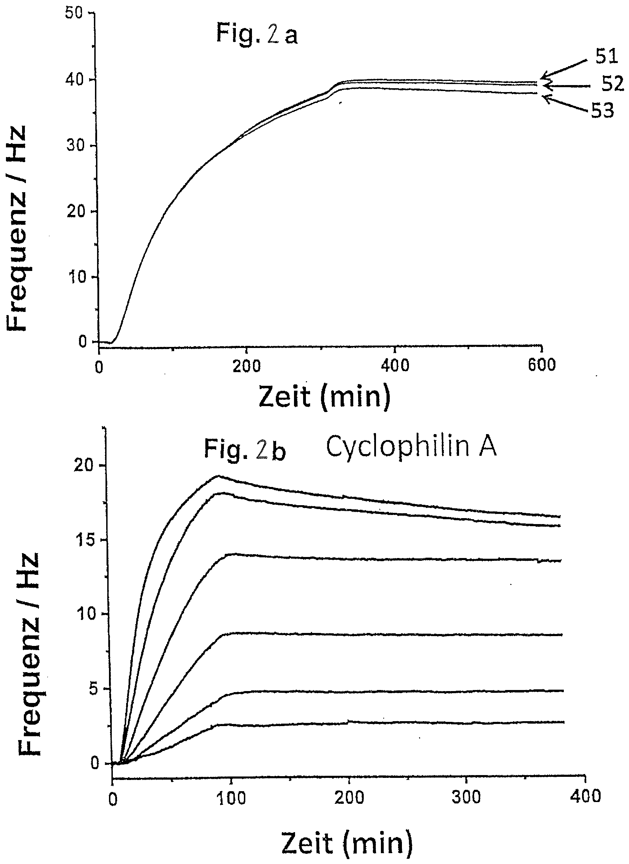

In Figur 2a ist eine, wie vorstehend beschriebene Hybridisierung von zwei DNA-Einzelsträngen (A';A) anhand eines QCM-Signal-Diagramms nachvollziehbar, wobei in einer mobilen Phase (Hybridisierungspuffer) vorliegende DNA-Einzelstränge A, die eine Konjugation mit Cyclosporin A (CsA) aufweisen, an immobilisierte DNA-Einstränge A' hybridisieren. Zum Lösen des durch die Hybridisierung immobilisierten zweiten Moleküls, d.h. der CsA-konjugierten DNA A, kann 2,5 mM HCl eingesetzt werden, mit der die Oberfläche des Sensorchips für 120 s überströmt bzw. inkubiert wird. Wie dem Diagramm der Figur 5a weiterhin zu entnehmen ist, zeigen Hybridisierungszyklen, d.h. wiederholte DNA-Kopplungen an die immobilisierten DNA-Einzelstränge A' 51, 52, und 53 korrespondierende QCM-Signale.

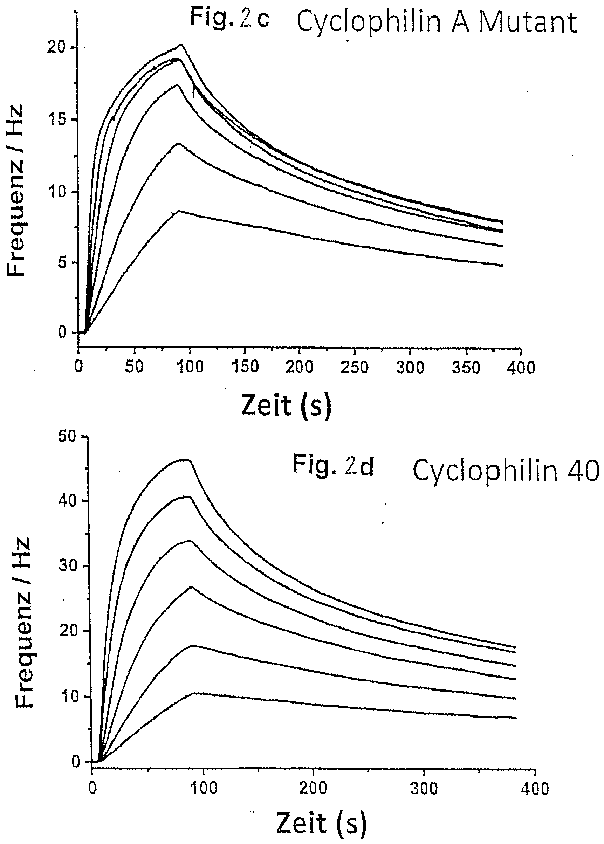

Mit den Figuren 2b bis 2d sind kinetische Experimente zur Bestimmung der Bindungsvorgänge (Assoziation und Dissoziation) zwischen den wie in Fig. 2a beschriebenen immobilisierten CsA Molekülen und Cyclophilin A (CypA, Fig. 2b) sowie die Varianten Cyclophilin A mutant (CypA mutant, Fig. 2c) und Cyclophilin 40 (Cyp40,Fig. 2d) anhand von QCM-Signal-Diagrammen dargestellt.

Zunächst wird der wie in Fig. 2a beschriebene QCM-Chip, auf dem die an DNA A gekoppelten CsA Moleküle immobilisiert sind, mit Protein-Bindungs-Puffer (10 mM HEPS, 150 mM NaCl, 0.05 % Tween 20, 1 µM BSA (bovines Serumalbumin), pH 7.4) gewaschen, wobei dem Protein-Bindungs-Puffer dabei jeweils Cyclophilin A (CypA, Fig. 2b), Cyclophilin A mutant (CypA mutant, Fig. 2c) und Cyclophilin 40 (Cyp40,Fig. 2d) in verschiedenen Konzentrationen zugegeben werden. Die entsprechenden Cyclophilin-Molekülkonzentrationen äußern sich in stärkeren QCM-Signalen, wie den Diagrammen der Figuren 2b bis 2d zu entnehmen ist. Die Injektionen (Kontaktierungen) der verschiedenen Molekülkonzentrationen des Cyclophilins A (CypA, Fig. 2b), Cyclophilins A mutant (CypA mutant, Fig. 2c) und Cyclophilins 40 (Cyp40, Fig. 2d) erfolgten jeweils bei einem Volumenstrom von 25µ/min bei 22°C für eine Injektionsdauer von 84 s.

Figur 2b zeigt die Anbindung und Dissoziation von CypA an dem QCM-Sensorchip im neunten Hybridisierungs- bzw. De-Hybridisierungszyklus. Dabei wurde CypA in folgenden Konzentrationen eingesetzt: 6.25 nM, 12.5 nM, 25 nM, 50 nM, 100 nM, und 200 nM. Nach Messung des QCM-Signals erfolgte jeweils eine Reinigung des Sensorchips durch Überströmen mit 1% Natriumlaurylsulfat-Puffer (SDS) für 120 s.

Figur 2c zeigt die Anbindung und Dissoziation von CypA mutant an dem QCM-Sensorchip, wobei das CypA mutant in den Konzentrationen 31.25 nM, 62.5 nM, 125 nM, 250 nM, 500 nM, und 1000 nM zugeführt wurde.

Figur 2d zeigt die Anbindung und Dissoziation von Cyp40 an dem QCM-Sensorchip, wobei das Cyp40 in den Konzentrationen 31.25 nM, 62.5 nM, 125 nM, 250 nM, 500 nM, und 1000 nM zugeführt wurde.

Bei keinem der in den Figuren 2b bis 2d verwendeten erfindungsgemäßen QCM-Sensorchips konnte während der Durchführung der Anbindungsexperimente (2b bis 2d) eine De-Hybridisierung der DNA Doppelhelix (A';A) infolge des Waschvorgangs mit der 1 % SDS-Pufferlösung festgestellt werden. Das Lösen bzw. die De-Hybridisierung des CsA gekoppelten DNA-Strangs A kann nur durch pH-Senkung erreicht werden.

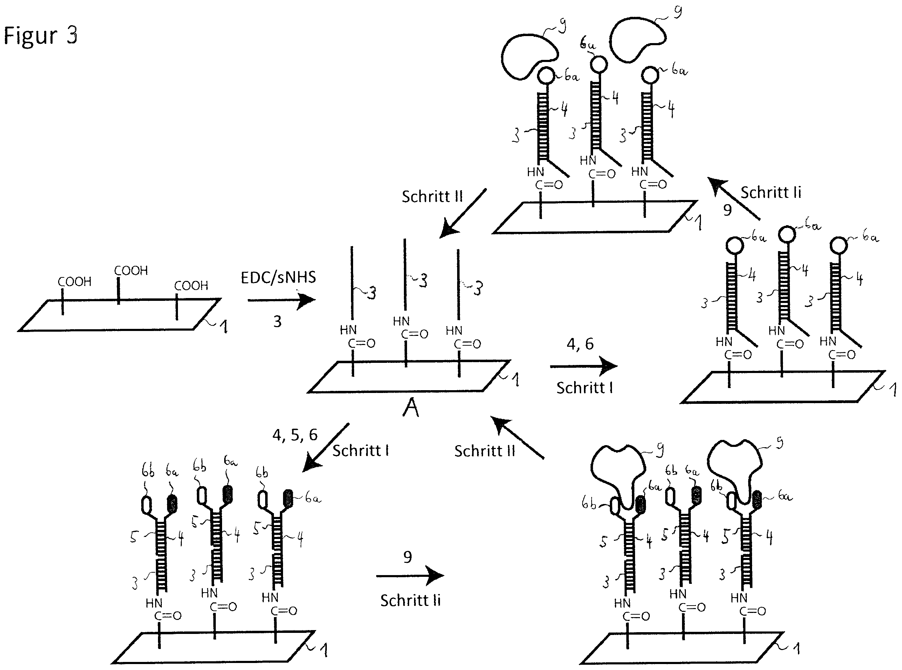

Die Figur 3 zeigt eine schematische Darstellung eines erfindungsgemäßen Verfahrensprinzips zur Erfassung von Bindungsereignissen von Molekülen an einer bioaktiven Oberfläche eines Biosensorchips, wobei mit den Pfeilen der Verfahrensfortschritt angedeutet ist. Im Ausgangszustand A ist die bioaktive Oberfläche 1 des Biosensorchips vollständig von einem Bindungspuffer bedeckt und weist eine Immobilisierung mindestens eines ersten Moleküls 3 auf, bei dem es sich um einzelsträngige DNA handelt, die chemisch kovalent an den Chip 1 gebunden ist. Diese chemisch kovalente Immobilisierung kann beispielsweise dadurch erreicht werden, indem ein Chip mit freien Carboxylgruppen mit einzelsträngiger DNA, die freie Aminogruppen aufweist (NH₂-ssDNA) in Gegenwart von EDC/sNHS zur Reaktion gebracht wird. In analoger Weise kann ein beliebiger Ligand 6a, 6b, der eine freie Carboxylgruppe aufweist, mit einer NH₂-ssDNA 4, 5 und EDC/sNHS zur Reaktion gebracht werden. Dadurch können erfindungsgemäße zweite Moleküle 4 und/oder dritte Moleküle 5 generiert werden, die chemisch kovalent einen Liganden 6a, 6b gebunden haben.

In einem Schritt (I) wird an dem mindestens einen ersten Molekül 3 zunächst ein zweites Molekül 4, bei dem es sich vorliegend um einzelsträngige DNA handelt, unter Bildung mindestens einer spezifischen nicht-kovalenten Bindung (Wasserstoffbrückenbindungen zwischen den Nukleotidbasen) gebunden (Hybridisierung). Das zweite Molekül 4 weist eine (z.B. chemisch kovalente) Bindung an einen Liganden 6a auf. Zusätzlich kann in Schritt (I) auch ein drittes Molekül 5 an das zweite Molekül 4 gebunden werden. Das dritte Molekül weist eine (z.B. chemisch kovalente) Bindung an einen (sog. zweiten) Liganden 6b auf, wobei die beiden Liganden 6a, 6b in diesem Beispiel verschieden sind.

Im Verfahrensschritt (Ii) werden die Moleküle mit Liganden durch Überströmen mit einer mobilen Phase kontaktiert, wobei in der mobilen Phase vorhandene Moleküle 9 (z.B. ein bestimmtes Protein als Analyt) an die Liganden 6a, 6b assoziieren können. Während des Verfahrensschritts (Ii) kann die Assoziation und Dissoziation zwischen den immobilisierten Liganden 6a, 6b und den Strukturen 9 analytisch ermittelt werden, beispielsweise mittels Oberflächenplasmonresonanz-Spektroskopie.

Im Schritt (II) wird die bioaktive Oberfläche 1 mit einer Pufferlösung mit einem pH-Wert kleiner als 3 durch Überströmen mit einem vorgebbaren Volumenstrom kontaktiert (nicht gezeigt). Durch den niedrigen pH-Wert wird die Hybridisierung der einzelsträngigen DNAs aufgelöst (Dissoziation) und das auf der Oberfläche 1 des Biochip chemisch kovalent immobilisierte erste Molekül 3 bleibt zurück d.h. der Biochip ist regeneriert und für eine erneute Assemblierung mit zweiten und optional dritten Molekülen bereit. Folglich ist das gesamte Verfahren reversibel, sodass der Biochip viele Male hintereinander für Messungen eingesetzt werden kann.

Im Anschluss an Schritt (II) kann die bioaktive Oberfläche 1 mit einem Laufpuffer (vorzugsweise Phosphatpuffer PBS mit pH 7) gespült werden, um den pH-Wert von pH 3 auf einen neutralen pH-Wert einzustellen.

Die Figur 2 zeigt eine schematische Darstellung eines Ausführungsbeispiels des erfindungsgemäßen Verfahrens anhand einer Beladung und Regenerierung eines Biosensorchips, bei dem der Biosensorchip im Ausgangszustand A eine Immobilisierung kovalent gebundener DNA Einzelstränge 3 als erstes Molekül aufweist. Dabei sind die DNA Einzelstränge 3 auf der bioaktiven Oberfläche 1 an kovalent gebundene Komplexe 2 kovalent immobilisiert. Mit dem Bezugszeichen 8 ist eine untere Phase gekennzeichnet, die zumindest aus den Komplexen 2 mit den daran kovalent gebundenen DNA Einzelsträngen 3 gebildet ist. In Schritt (I) des Verfahrens wird die untere Phase 8 mit einer Pufferlösung kontaktiert, in der zweite DNA Einzelstränge 4 als zweites Molekül enthalten sind. Dabei weisen die zweiten DNA Einzelstränge 4 kovalent gekoppelte Liganden 6 auf. Während der Kontaktierung hybridisieren die zweiten DNA Einzelstränge 4 komplementär, d.h. spezifisch nicht-kovalent an die immobilisierten DNA Einzelstränge 3 unter Bildung von DNA Doppelsträngen. Mit dem Bezugszeichen 7 ist dabei der Bereich dargestellt, der die obere stationäre Phase bildet.

Im Verfahrensschritt (Ii) wird die stationäre Phase 7 durch Überströmen mit einer mobilen Phase kontaktiert, wobei in der mobilen Phase vorhandene Strukturen 9 (Analyten) an den Liganden 6 assoziieren können. Während des Verfahrensschritts (Ii) kann die Assoziation und Dissoziation beispielsweise mittels Quarzkristall-Mikrowaagen ermittelt werden.

Zur Regeneration (Ablösen der temporären stationären Phase, Bezugszeichen 7) wird die bioaktive Oberfläche 1 in Schritt (II) mit einer Pufferlösung durch Überströmen mit einer vorgebbaren Strömungsgeschwindigkeit kontaktiert, wobei die Pufferlösung anfänglich einen pH Wert aufweist, der im Bereich von pH 8 bis pH 6 liegt und während des Kontaktierens bis zum Erreichen eines pH-Werts kleiner als 3 langsam verringert wird.

Infolge der pH-Wert Änderung während der Kontaktierung mit der Pufferlösung denaturieren die aus den DNA Einzelsträngen 3 und 4 gebildeten Doppelstränge, wobei die zweiten DNA Einzelstränge 4 von den ersten immobilisierten DNA Einzelsträngen 3 dissoziieren. Dabei wird die stationäre Phase 7 gelöst und mit der Strömung der Pufferlösung abgeführt. Abschließend wird ein Neutralisationspuffer oder Laufpuffer zugeführt, der einen physiologischen pH-Wert im Bereich von pH 6 bis pH 7 aufweist, wobei die immobilisierten DNA Einzelstränge 3 renaturieren können.

Die Figur 3 zeigt ein Ausführungsbeispiel der erfindungsgemäßen Anordnung zur Erfassung von Bindungsereignissen von Molekülen, mit mindestens einem ersten Molekül 3, das an einer bioaktiven Oberfläche 1 durch eine kovalente Bindung 20 immobilisiert ist. Dabei bildet das kovalent gebundene erste Molekül 3 eine untere Phase 8. Weiterhin ist mit dem Bezugszeichen 4 ein zweites Molekül gezeigt, das durch eine spezifische nicht-kovalente Bindung an dem mindestens einen ersten Molekül 3 gebunden ist. Bei dem ersten Molekül 3 handelt es sich im vorliegenden Beispiel um das Biotin-Derivat Desthiobiotin und bei dem zweiten Molekül 4 um Streptavidin, wobei das Desthiobiotin 3 zu dem Streptavidin 4 eine Dissoziationskonstante K_{D} im Bereich von 10⁻⁶ M bis 10⁻¹⁰ M aufweist.

An dem Streptavidin 4, das eine geschlossene Schicht bildet, sind weitere dritte Moleküle 5 gebunden, die eine Kopplung eines Liganden 6 aufweisen. Im vorliegenden Beispiel handelt es sich bei den Molekülen 5 um Biotin-Moleküle, an denen die Liganden 6 kovalent gebunden sind.

Die mit dem Streptavidin 4 und dem biotinylierten (Biotin-Moleküle enthaltende) Liganden 5/6 gebildete obere stationäre Phase 7 kann durch das Lösen der spezifischen nicht-kovalenten Bindung zwischen dem Desthiobiotin 3 und dem Streptavidin 4 von der bioaktiven Oberfläche 1 gelöst werden.

Die zwischen dem Desthiobiotin 3 und dem Streptavidin 4 gebildete spezifische nicht-kovalente Bindung wird gelöst, in dem die bioaktive Oberfläche 1 mit einer Pufferlösung kontaktiert wird, die freie ungebundene Biotin-Moleküle enthält. Die Dissoziationskonstante von Biotin und dem Streptavidin 4 liegt unterhalb von 10⁻¹⁴ M, so dass die freien Biotin-Moleküle eine stärkere Affinität zur spezifischen Bindung von Streptavidin haben, als das Desthiobiotin 3. Folglich verdrängen die zugeführten Biotin-Moleküle das Desthiobiotin 3 von den Bindungsstellen des Streptavidins 4, wobei die obere stationäre Phase 7 von der unteren Phase 8 gelöst wird. Je nach Messmethode (bspw. SPR, LIM oder QCM) kann die erfindungsgemäße Anordnung entsprechend ausgebildet sein. So kommt die erfindungsgemäße Anordnung bspw. in Verbindung mit SPR- LIM- oder QCM-Sensorchips oder -bioassays in Frage. Ist die erfindungsgemäße Anordnung auf und/oder mit Nano-Partikeln oder Beads realisiert, sind zusätzlich zu den genannten Verfahren SPR, QCM oder LIM (Lichtinterferenzmessung) auch andere Messverfahren/Nachweisverfahren denkbar, die auf dem Prinzip der fluoreszenzaktivierten Partikelsortierung basieren. Es versteht sich, dass in diesem Fall geeignete Fluoreszenzmarker (Markierungen) zum Einsatz kommen, die spezifisch an mindestens ein auf der bioaktiven Oberfläche gebundenes oder immobilisiertes Molekül binden.

Nachfolgend wird die Herstellung bzw. Bereitstellung von Amino-PEG-Desthiobiotin, das sich wegen seiner Amino-Funktionalisierung und PEG(Polyethylenglycol)ylierung insbesondere zur Anbindung an immobilisierte Diamino-Linker und somit zur Bildung der unteren Phase (Figur 1 Bezugszeichen 8) eignet, an einem Beispiel näher erläutert.

Zur Herstellung von Amino-PEG-Desthiobiotin werden zunächst 107.5 mg Desthiobiotin mit 500 mg 0-Bis-(Aminoethyl)ethylen-Glykol-Trityl-Harz, unter Verwendung von 165 µl N-methylmorpholin und 170 mg HBTU (Peptid-Bindungs-Reagenz) in 2 ml Dimethylformamid (DMF) zusammen gebracht und für 30 min geschüttelt. Anschließend wird das Harz mit DMF und Dichlormethan (DCM) gewaschen. Zur Abtrennung wird das Harz für 20 min mit 3 ml einer Inkubationslösung, bestehend aus 3 ml DCM und 2 % Trifluoressigsäure (TFA), inkubiert. Nachfolgend wird das Harz zweimal mit jeweils 1 ml der Inkubationslösung gewaschen. Im nächsten Schritt erfolgt eine Ausfällung des abgetrennten Produkts mit bzw. in 30 ml Diethylether sowie die Filterung des Präzipitats mit einem 0,22 µm Filter. Anschließend wird das Retentat in Wasser gelöst und via RP-HPLC aufgereinigt. Hierzu kann beispielsweise eine RP-HPLC ProStar, Agilent Technologies, Santa Clara, Unites States verwendet werden, die mit einer C18 Säule von AXIA 100A, bead size 10µm, 250x30 mm, Phenomenex, Torrance, Unites States, ausgestattet ist, wobei die Säule für 50 min mit einem linearen Gradienten beginnend bei 5 % bis 95 % eluiert wird. Der Gradient wird durch zwei Lösungen A und B bereitgestellt, von denen die Lösung A 99.9 % Wasser und 0.1 % TFA und die Lösung B 94.9 % Acetonitril, 5 % Wasser und 0.1 % TFA enthält. Zur Bestimmung der gesuchten Fraktion des Amino-PEG-Desthiobiotins kann beispielsweise ESI-MS eingesetzt werden. Die Fraktion des Amino-PEG-Desthiobiotins kann als Lyophilisat in einem Öl aufgenommen werden.

Nachfolgend wird an einem weiteren Beispiel dargestellt, wie das Amino-PEG-Desthiobiotin als erstes Molekül 3 zur Bildung der unteren Phase der erfindungsgemäßen Anordnung insbesondere zur Ausbildung der bioaktiven Oberfläche eingesetzt werden kann bzw. einsetzbar ist. Als Basis dient dabei eine bioaktive Oberfläche eines Carboxyl-Sensor-Chips.

Zunächst wird ein Carboxyl-Sensor-Chip (Sensorchip dessen Oberfläche mit Carboxylgruppen beladen ist) mit 10 µm/min Laufpuffer (1-fach PBS, pH 7,4) äquilibriert. Anschließend erfolgt eine Inkubation des Sensorchips für 300 s mit einer Lösung bestehend aus 0.2 M 1-Ethyl-3-[3 dimethylaminopropyl]Carbodiimid-Hydrochlorid (EDC) und 0.05 M N-hydroxysulfosuccinimid (sulfo-NHS). Bei der Inkubation des Sensorchips werden die Carboxylgruppen derart funktionalisiert, dass sie zur Bindung bzw. Kopplung des Amino-PEG-Desthiobiotins fähig sind. Entsprechend wird die Oberfläche des Sensorchips in einer weiteren Inkubation für 300 s dem Amino-funktionalisierten PEGylierten Desthiobiotin (2 µl Öl des Amino-PEG-Desthiobiotins in 300 µl 10 mM Acetatpuffer, pH 4.75) ausgesetzt. Nach der Inkubation werden die verbleibenden aktivierten Carboxylgruppen mit 1 M (pH 8) Ethanolamin geblockt. Abschließend wird der Sensorchip mit 25 µl/min Laufpuffer äquilibriert. Das Ergebnis entspricht einem wie in Figur 1 im Zustand A gekennzeichneten Biosensorchip, auf dessen Oberfläche 1 die untere Phase 8 gebildet ist, wobei das Bezugszeichen 3 das Amino-PEG-Desthiobiotin darstellt. Die so funktionalisierte Oberfläche 1 des Sensorchips ist zur reversiblen Anbindung weiterer zweiter Moleküle 4, vorzugsweise Streptavidin geeignet.

In Figur 4 ist die reversible Anbindung des Streptavidins an einem mit Amino-PEG-Desthiobiotin funktionaliserten Sensorchip anhand eines QCM-Diagramms beispielhaft dargestellt. Es handelt sich dabei folglich um eine Anwendung der erfindungsgemäßen Vorrichtung mit einem QCM-Sensorchip. Nach Äquilibrierung mit 25 µl/min Laufpuffer wird der mit Amino-PEG-Desthiobiotin funktionalisierte Sensorchip für 300 s mit Streptavidin kontaktiert. In Figur 4 sind diese 300 s mit dem Abschnitt zwischen den Bezugszeichen 41 und 42 dargestellt. Die Kontaktierung mit Streptavidin erzeugt dabei ein starkes QCM-Signal (Y-Achse in Hz).

Nach der Kontaktierung mit Streptavidin wird die bioaktive Oberfläche des Sensorchips mit Laufpuffer äquilibriert, wobei ungebundenes Streptavidin mit dem Flüssigkeitsstrom des Laufpuffers abgeführt wird. Die Äquilibierung wird beendet, wenn sich das QCM-Signal im Wesentlichen nicht mehr ändert, so dass davon ausgegangen werden kann, dass auf der Oberfläche des Sensorchips ausschließlich an das Amino-PEG-Desthiobiotin gebundene Streptavidinmoleküle verblieben sind. Im vorliegenden Fall wurde die Äquilibrierung mit dem Laufpuffer nach ca. 51 min beendet (Bezugszeichen 43), da ein konstantes QCM-Signal gemessen werden konnte. An der mit dem Bezugszeichen 43 gekennzeichneten Position ist der Sensorchip bzw. die Oberfläche des Sensorchips geeignet, weitere dritte, insbesondere biotinylierte Moleküle bzw. Liganden zu binden. Für solche kinetischen Experimente (Assoziation und Dissoziation von Liganden und ihren Analyten) kann das Streptavidin auch zuvor, d.h. vor der Anbindung an den Sensorchip mit biotinylierten chem. Verbindungen bzw. biotinylierten Komponenten (dritten Liganden/Molekülen) im Verhältnis 2:1 (Komponente zu Streptavidin) inkubiert werden.

Im nachfolgenden Schritt (II) erfolgt die Regeneration der bioaktiven Oberfläche, wobei unter Regeneration das Lösen des zweiten Moleküls, d.h. das Lösen des Streptavidins verstanden wird. Zum Ablösen (Dissoziation) des Streptavidins wird die bioaktive Oberfläche des Sensorchips dreimal für jeweils 300 s mit 1 mg/ml Biotin in PBS kontaktiert, wobei jeweils ein Volumenstrom von 25µl/min eingehalten wird, so dass abgelöste Moleküle abgeführt werden können. Der Vorgang des Ablösens kann anhand der mit den Bezugszeichen 43-44, 44-45, 45-46 bezeichneten Abschnitte nachvollzogen werden, wobei das QCM-Signal stetig kleiner wird. Nach dem Ablösen erfolgt eine weitere Beladung mit Streptavidin, wobei die bioaktive Oberfläche des Sensorchips ein weiteres Mal für 300 s mit Streptavidin kontaktiert wird. Die erneute Beladung kann anhand des im Abschnitt 46-47 sprunghaft ansteigenden QCM-Signals nachvollzogen werden. Nach der Kontaktierung mit Streptavidin wird die Oberfläche des Sensorchips mit Laufpuffer äquilibriert, um unspezifisch gebundenes bzw. ungebundenes Streptavidin mit dem Volumenstrom des Laufpuffers abzuführen. Wie mit dem Bezugszeichen 48 dargestellt, wird auch bei wiederholter Streptavidin Beladung eine Streptavidinanbindung erreicht, die der zuvor vorgenommenen Streptavidinanbindung an Position 43 entspricht.

Das erfindungsgemäße Verfahren wird nachfolgend an einem weiteren Ausführungsbeispiel anhand eines QCM-DNA-Chips (Attana A200^{®} System) erläutert.

In einem ersten Schritt wird der QCM-Chip, auf dem als erstes Molekül DNA Einzelstränge A' immobilisiert sind, mit einem Laufpuffer pH 7,4 bestehend aus 10 mM HEPS (Puffersubstanz), 150 mM NaCl und 0,05 % TWEEN 20 gespült bzw. gewaschen. Nachfolgend werden 1µM in Hybridisierungspuffer vorliegende (komplementäre) DNA Einzelstränge A, die mit organischen Molekülen (Liganden z.B. Cyclosporin A) konjugiert (kovalent gebunden) sind, mit der Substratoberfläche, d.h. mit den DNA Einzelsträngen A' kontaktiert. Der hierzu verwendete Hybridisierungspuffer weist pH 7.4 auf und kann beispielsweise 10 mM HEPS sowie 150 mM NaCl enthalten.

In Figur 5a ist eine, wie vorstehend beschriebene Hybridisierung von zwei DNA-Einzelsträngen (A';A) anhand eines QCM-Signal-Diagramms nachvollziehbar, wobei in einer mobilen Phase (Hybridisierungspuffer) vorliegende DNA-Einzelstränge A, die eine Konjugation mit Cyclosporin A (CsA) aufweisen, an immobilisierte DNA-Einstränge A' hybridisieren. Zum Lösen des durch die Hybridisierung immobilisierten zweiten Moleküls, d.h. der CsA-konjugierten DNA A, kann 2,5 mM HCl eingesetzt werden, mit der die Oberfläche des Sensorchips für 120 s überströmt bzw. inkubiert wird. Wie dem Diagramm der Figur 5a weiterhin zu entnehmen ist, zeigen Hybridisierungszyklen, d.h. wiederholte DNA-Kopplungen an die immobilisierten DNA-Einzelstränge A' 51, 52, und 53 korrespondierende QCM-Signale.

Mit den Figuren 5b bis 5d sind kinetische Experimente zur Bestimmung der Bindungsvorgänge (Assoziation und Dissoziation) zwischen den wie in Fig. 5a beschriebenen immobilisierten CsA Molekülen und Cyclophilin A (CypA, Fig. 5b) sowie die Varianten Cyclophilin A mutant (CypA mutant, Fig. 5c) und Cyclophilin 40 (Cyp40,Fig. 5d) anhand von QCM-Signal-Diagrammen dargestellt.

Zunächst wird der wie in Fig.5a beschriebene QCM-Chip, auf dem die an DNA A gekoppelten CsA Moleküle immobilisiert sind, mit Protein-Bindungs-Puffer (10 mM HEPS, 150 mM NaCl, 0.05 % Tween 20, 1 µM BSA (bovines Serumalbumin), pH 7.4) gewaschen, wobei dem Protein-Bindungs-Puffer dabei jeweils Cyclophilin A (CypA, Fig. 5b), Cyclophilin A mutant (CypA mutant, Fig. 5c) und Cyclophilin 40 (Cyp40,Fig. 5d) in verschiedenen Konzentrationen zugegeben werden. Die entsprechenden Cyclophilin-Molekülkonzentrationen äußern sich in stärkeren QCM-Signalen, wie den Diagrammen der Figuren 5b bis 5d zu entnehmen ist. Die Injektionen (Kontaktierungen) der verschiedenen Molekülkonzentrationen des Cyclophilins A (CypA, Fig. 5b), Cyclophilins A mutant (CypA mutant, Fig. 5c) und Cyclophilins 40 (Cyp40,Fig. 5d) erfolgten jeweils bei einem Volumenstrom von 25µl/min bei 22°C für eine Injektionsdauer von 84 s.

Figur 5b zeigt die Anbindung und Dissoziation von CypA an dem QCM-Sensorchip im neunten Hybridisierungs- bzw. De-Hybridisierungszyklus. Dabei wurde CypA in folgenden Konzentrationen eingesetzt: 6.25 nM, 12.5 nM, 25 nM, 50 nM, 100 nM, und 200 nM. Nach Messung des QCM-Signals erfolgte jeweils eine Reinigung des Sensorchips durch Überströmen mit 1% Natriumlaurylsulfat-Puffer (SDS) für 120 s.

Figur 5c zeigt die Anbindung und Dissoziation von CypA mutant an dem QCM-Sensorchip, wobei das CypA mutant in den Konzentrationen 31.25 nM, 62.5 nM, 125 nM, 250 nM, 500 nM, und 1000 nM zugeführt wurde.

Figur 5d zeigt die Anbindung und Dissoziation von Cyp40 an dem QCM-Sensorchip, wobei das Cyp40 in den Konzentrationen 31.25 nM, 62.5 nM, 125 nM, 250 nM, 500 nM, und 1000 nM zugeführt wurde.

Bei keinem der in den Figuren 5b bis 5d verwendeten erfindungsgemäßen QCM-Sensorchips konnte während der Durchführung der Anbindungsexperimente (5b bis 5d) eine De-Hybridisierung der DNA Doppelhelix (A';A) infolge des Waschvorgangs mit der 1 % SDS-Pufferlösung festgestellt werden. Das Lösen bzw. die De-Hybridisierung des CsA gekoppelten DNA-Strangs A kann nur durch pH-Senkung erreicht werden.

Die Figur 6 zeigt eine schematische Darstellung eines weiteren erfindungsgemäßen Verfahrensprinzips zur Erfassung von Bindungsereignissen von Molekülen an einer bioaktiven Oberfläche eines Biosensorchips, wobei mit den Pfeilen der Verfahrensfortschritt angedeutet ist. Im Ausgangszustand A ist die bioaktive Oberfläche 1 des Biosensorchips vollständig von einem Bindungspuffer bedeckt und weist eine Immobilisierung mindestens eines ersten Moleküls 3 auf, bei dem es sich um einzelsträngige DNA handelt, die chemisch kovalent an den Chip 1 gebunden ist. Diese chemisch kovalente Immobilisierung kann beispielsweise dadurch erreicht werden, indem ein Chip mit freien Carboxylgruppen mit einzelsträngiger DNA, die freie Aminogruppen aufweist (NH₂-ssDNA) in Gegenwart von EDC/sNHS zur Reaktion gebracht wird. In analoger Weise kann ein beliebiger Ligand 6a, 6b, der eine freie Carboxylgruppe aufweist, mit einer NH₂-ssDNA 4, 5 und EDC/sNHS zur Reaktion gebracht werden. Dadurch können erfindungsgemäße zweite Moleküle 4 und/oder dritte Moleküle 5 generiert werden, die chemisch kovalent einen Liganden 6a, 6b gebunden haben.

In einem Schritt (I) wird an dem mindestens einen ersten Molekül 3 zunächst ein zweites Molekül 4, bei dem es sich vorliegend um einzelsträngige DNA handelt, unter Bildung mindestens einer spezifischen nicht-kovalenten Bindung (Wasserstoffbrückenbindungen zwischen den Nukleotidbasen) gebunden (Hybridisierung). Das zweite Molekül 4 weist eine (z.B. chemisch kovalente) Bindung an einen Liganden 6a auf. Zusätzlich kann in Schritt (I) auch ein drittes Molekül 5 an das zweite Molekül 4 gebunden werden. Das dritte Molekül weist eine (z.B. chemisch kovalente) Bindung an einen (sog. zweiten) Liganden 6b auf, wobei die beiden Liganden 6a, 6b in diesem Beispiel verschieden sind.

Im Verfahrensschritt (Ii) werden die Moleküle mit Liganden durch Überströmen mit einer mobilen Phase kontaktiert, wobei in der mobilen Phase vorhandene Moleküle 9 (z.B. ein bestimmtes Protein als Analyt) an die Liganden 6a, 6b assoziieren können. Während des Verfahrensschritts (Ii) kann die Assoziation und Dissoziation zwischen den immobilisierten Liganden 6a, 6b und den Strukturen 9 analytisch ermittelt werden, beispielsweise mittels Oberflächenplasmonresonanz-Spektroskopie.

Im Schritt (II) wird die bioaktive Oberfläche 1 mit einer Pufferlösung mit einem pH-Wert kleiner als 3 durch Überströmen mit einem vorgebbaren Volumenstrom kontaktiert (nicht gezeigt). Durch den niedrigen pH-Wert wird die Hybridisierung der einzelsträngigen DNAs aufgelöst (Dissoziation) und das auf der Oberfläche 1 des Biochip chemisch kovalent immobilisierte erste Molekül 3 bleibt zurück d.h. der Biochip ist regeneriert und für eine erneute Assemblierung mit zweiten und optional dritten Molekülen bereit. Folglich ist das gesamte Verfahren reversibel, sodass der Biochip viele Male hintereinander für Messungen eingesetzt werden kann.

Im Anschluss an Schritt (II) kann die bioaktive Oberfläche 1 mit einem Laufpuffer (vorzugsweise Phosphatpuffer PBS mit pH 7) gespült werden, um den pH-Wert von pH 3 auf einen neutralen pH-Wert einzustellen.

## Patentansprüche

1. Verfahren zur Erfassung von Bindungsereignissen von Molekülen an bioaktiven Oberflächen, bei dem
in einem Schritt (I),
an mindestens ein erstes Molekül (3), das durch eine kovalente Bindung an einer bioaktiven Oberfläche (1) immobilisiert ist, wobei das erste Molekül (3) ausgewählt ist aus der Gruppe bestehend aus einzelsträngiger DNA, RNA, LNA und PNA,
mindestens ein zweites Molekül (4) unter Bildung mindestens einer spezifischen nicht-kovalenten Bindung gebunden wird, wobei das mindestens eine zweite Molekül (4) ausgewählt ist aus der Gruppe bestehend aus einzelsträngiger DNA, RNA, LNA und PNA und das zweite Molekül (4) chemisch kovalent an einen Liganden (6a) gebunden ist oder wird,
eine Anbindung mindestens eines dritten Moleküls (5) an das mindestens eine zweite Molekül (4) über mindestens eine spezifische nicht-kovalente Bindung erreicht wird, wobei das mindestens eine dritte Molekül (5) ausgewählt ist aus der Gruppe bestehend aus einzelsträngiger DNA, RNA, LNA und PNA und das mindestens eine dritte Molekül (5) kovalent an einen zweiten Liganden (6b) gebunden ist oder wird,
eine erste Pufferlösung enthaltend ein Analytmolekül (9) mit der Oberfläche (1) kontaktiert wird und mittels einer analytischen Messmethode Bindungsereignisse zwischen dem Analytmolekül (9) und dem Liganden (6a) und zweiten Liganden (6b) erfasst werden,
in einem Schritt (II),
eine zweite Pufferlösung mit der Oberfläche (1) kontaktiert wird, sodass die spezifische nicht-kovalente Bindung zwischen dem ersten Molekül (3) und dem zweiten Molekül (4) gelöst wird und nicht-kovalent an der Oberfläche gebundene Moleküle von der Oberfläche entfernt werden, und
im Anschluss daran die Schritte (I) und (II) mindestens einmal wiederholt werden.

2. Verfahren nach Anspruch 1, **dadurch gekennzeichnet, dass** der Ligand (6a) ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

3. Verfahren nach einem der vorhergehenden Ansprüche, **dadurch gekennzeichnet, dass** der zweite Ligand (6b) ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

4. Verfahren nach einem der vorhergehenden Ansprüche, **dadurch gekennzeichnet, dass** der Ligand (6a) und der zweite Ligand (6b) identisch oder unterschiedlich sind.

5. Verfahren nach einem der vorhergehenden Ansprüche, **dadurch gekennzeichnet, dass** die Bindungsereignisse mittels Oberflächenplasmonresonanz-Spektroskopie (SPR), Lichtinterferenzmessung (LIM) und/oder Quarzkristall-Mikrowaagen (QCM) erfasst werden.

6. Verfahren nach einem der vorhergehenden Ansprüche, **dadurch gekennzeichnet, dass** das mindestens eine Analytmolekül (9) ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

7. Verfahren nach einem der vorhergehenden Ansprüche, **dadurch gekennzeichnet, dass** die bioaktive Oberfläche (1) im Schritt (II) mit der Pufferlösung kontaktiert wird, wobei die Pufferlösung einen pH-Wert kleiner als 3 aufweist.

8. Verfahren nach einem der vorhergehenden Ansprüche, **dadurch gekennzeichnet, dass** die bioaktive Oberfläche (1) nach dem Lösen der spezifischen nicht-kovalenten Bindung mit einem Neutralisationspuffer kontaktiert wird, der einen pH-Wert im Bereich von pH 6 bis pH 8 aufweist.

9. Anordnung zur Erfassung von Bindungsereignissen von Molekülen, bei der
mindestens ein erstes Molekül (3) an einer bioaktiven Oberfläche (1) durch eine kovalente Bindung immobilisiert ist, wobei das erste Molekül (3) ausgewählt ist aus der Gruppe bestehend aus einzelsträngiger DNA, RNA, LNA und PNA, und
mindestens ein zweites Molekül (4) durch eine spezifische nicht-kovalente Bindung an dem mindestens einen ersten Molekül (3) gebunden ist, wobei das mindestens eine zweite Molekül (4) ausgewählt ist aus der Gruppe bestehend aus einzelsträngiger DNA, RNA, LNA und PNA und das zweite Molekül (4) chemisch kovalent an einen Liganden (6a) gebunden ist, wobei
mindestens ein drittes Molekül (5) über mindestens eine spezifische nicht-kovalente Bindung an das mindestens eine zweite Molekül (4) angebunden ist, wobei das mindestens eine dritte Molekül (5) ausgewählt ist aus der Gruppe bestehend aus einzelsträngiger DNA, RNA, LNA und PNA und das mindestens eine dritte Molekül (5) kovalent an einen zweiten Liganden (6b) gebunden ist, wobei
die spezifische nicht-kovalente Bindung der ersten und zweiten Moleküle (3, 4) durch Kontaktieren der Oberfläche (1) mit einer Pufferlösung lösbar ist.

10. Anordnung nach Anspruch 9, **dadurch gekennzeichnet, dass** der Ligand (6a) ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

11. Anordnung nach einem der Ansprüche 9 oder 10, **dadurch gekennzeichnet, dass** der zweite Ligand (6b) ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

12. Anordnung nach einem der Ansprüche 9 bis 11, **dadurch gekennzeichnet, dass** der Ligand (6a) und der zweite Ligand (6b) identisch oder unterschiedlich sind.

13. Anordnung nach einem der Ansprüche 9 bis 12, **dadurch gekennzeichnet, dass** die bioaktive Oberfläche (1) für eine analytischen Messung von Bindungsereignissen geeignet ist, bevorzugt für eine analytische Messung mittels Oberflächenplasmonresonanz-Spektroskopie (SPR), Lichtinterferenzmessung (LIM) und/oder Quarzkristall-Mikrowaagen (QCM).

14. Anordnung nach einem der Ansprüche 9 bis 13, **dadurch gekennzeichnet, dass** mindestens ein Analytmolekül (9), bevorzugt über eine nicht-kovalente Bindung, an die bioaktive Oberfläche (1) gebunden ist, wobei das Analytmolekül bevorzugt ausgewählt ist aus der Gruppe bestehend aus Peptiden, Proteinen, Lipiden, DNA, RNA, PNA, LNA, Oligosacchariden sowie niedermolekularen Verbindungen mit einer Molekülmasse von ≤ 20.000 Da, bevorzugt ≤ 10.000 Da, besonders bevorzugt ≤ 3000 Da, insbesondere ≤ 900 Da.

15. Anordnung nach einem der Ansprüche 9 bis 14, **dadurch gekennzeichnet, dass** die spezifische nicht kovalente Bindung bei Kontaktierung mit einer Pufferlösung, die einen pH-Wert aufweist der kleiner ist als pH 3, ablösbar ist.

16. Anordnung nach einem der Ansprüche 9 bis 15, **dadurch gekennzeichnet, dass** die Oberfläche (1) im Wesentlichen trocken ist.

## Claims

1. Method for detecting binding events of molecules on bioactive surfaces, in which
in a step (I),
to at least one first molecule (3), which is immobilised on a bioactive surface (1) by a covalent bond, the first molecule (3) being selected from the group consisting of single-strand DNA, RNA, LNA and PNA,
at least one second molecule (4) is bonded with formation of at least one specific non-covalent bond, the at least one second molecule (4) being selected from the group consisting of single-strand DNA, RNA, LNA and PNA and the second molecule (4) being bonded or having been bonded chemically covalently to a ligand (6a),
a binding of at least one third molecule (5) to the at least one second molecule (4) via at least one specific non-covalent bond is achieved, the at least one third molecule (5) being selected from the group consisting of single-strand DNA, RNA, LNA and PNA, and the at least one third molecule (5) being bonded or having been bonded covalently to a second ligand (6b),
a first buffer solution comprising an analyte molecule (9) is contacted with the surface (1) and the binding events between the analyte molecule (9) and the ligand (6a) and second ligand (6b) are detected by means of an analytical measuring method,
in a step (II),
a second buffer solution is contacted with the surface (1) so that the specific non-covalent bond between the first molecule (3) and the second molecule (4) is broken and molecules which are bonded non-covalently on the surface are removed from the surface, and subsequently thereto, steps (I) and (II) are repeated at least once.

2. Method according to claim 1, **characterised in that** the ligand (6a) is selected from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

3. Method according to one of the preceding claims, **characterised in that** the second ligand (6b) is selected from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

4. Method according to one of the preceding claims, **characterised in that** the ligand (6a) and the second ligand (6b) are identical or different.

5. Method according to one of the preceding claims, **characterised in that** the binding events are detected by means of surface plasmon resonance spectroscopy (SPR), light interference measurement (LIM) and/or quartz crystal microbalances (QCM).

6. Method according to one of the preceding claims, **characterised in that** the at least one analyte molecule (9) is selected from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

7. Method according to one of the preceding claims, **characterised in that** the bioactive surface (1) is contacted with the buffer solution in step (II), the buffer solution having a pH value less than 3.

8. Method according to one of the preceding claims, **characterised in that** the bioactive surface (1), after breaking the specific non-covalent bond, is contacted with a neutralisation buffer which has a pH value in the range of pH 6 to pH 8.

9. Arrangement for detecting binding events of molecules, in which
at least one first molecule (3) is immobilised on a bioactive surface (1) by a covalent bond, the first molecule (3) being selected from the group consisting of single-strand DNA, RNA, LNA and PNA, and
at least one second molecule (4) is bonded by a specific non-covalent bond to the at least one first molecule (3), the at least one second molecule (4) being selected from the group consisting of single-strand DNA, RNA, LNA and PNA, and the second molecule (4) being bonded chemically covalently to a ligand (6a),
at least one third molecule (5) being bonded to the at least one second molecule (4) via at least one specific non-covalent bond, the at least one third molecule (5) being selected from the group consisting of single-strand DNA, RNA, LNA and PNA, and the at least one third molecule (5) being bonded covalently to a second ligand (6b),
the specific non-covalent bond of the first and second molecules (3, 4) being breakable by contacting the surface (1) with a buffer solution.

10. Arrangement according to claim 9, **characterised in that** the ligand (6a) is selected from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

11. Arrangement according to one of the claims 9 or 10, **characterised in that** the second ligand (6b) is selected from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

12. Arrangement according to one of the claims 9 to 11, **characterised in that** the ligand (6a) and the second ligand (6b) are identical or different.

13. Arrangement according to one of the claims 9 to 12, **characterised in that** the bioactive surface (1) is suitable for an analytical measurement of binding events, preferably for an analytical measurement by means of surface plasmon resonance spectroscopy (SPR), light interference measurement (LIM) and/or quartz crystal microbalances (QCM).

14. Arrangement according to one of the claims 9 to 13, **characterised in that** at least one analyte molecule (9) is bonded to the bioactive surface (1), preferably via a non-covalent bond, the analyte molecule being selected preferably from the group consisting of peptides, proteins, lipids, DNA, RNA, PNA, LNA, oligosaccharides and also low-molecular compounds with a molecular mass of ≤ 20,000 Da, preferably ≤ 10,000 Da, particularly preferably ≤ 3,000 Da, in particular ≤ 900 Da.

15. Arrangement according to one of claims 9 to 14, **characterised in that** the specific non-covalent bond is breakable by contacting with a buffer solution which has a pH value which is less than pH 3.

16. Arrangement according to one of the claims 9 to 15, **characterised in that** the surface (1) is essentially dry.

## Revendications

1. Procédé pour détecter des événements de liaison moléculaire sur des surfaces bioactives, dans lequel
lors d'une première étape (I),
à au moins une première molécule (3), qui par une liaison covalente est immobilisée sur une surface bioactive (1), la première molécule (3) étant choisie dans le groupe consistant en l'ADN, l'ARN, le LNA ou le PNA simple brin,
on lie une deuxième molécule (4), avec formation d'au moins une liaison non covalente spécifique, l'au moins une deuxième molécule (4) étant choisie dans le groupe consistant en l'ADN, l'ARN, le LNA et le PNA simple brin, et la deuxième molécule (4) étant liée, ou mise en liaison chimiquement covalente avec un ligand (6a),
on réalise une liaison d'au moins une troisième molécule (5) à l'au moins une deuxième molécule (4) par l'intermédiaire d'au moins une liaison non covalente spécifique, l'au moins une troisième molécule (5) étant choisie dans le groupe consistant en l'ADN, l'ARN, le LNA et le PNA simple brin, et l'au moins une troisième molécule étant liée ou mise en liaison covalente avec un deuxième ligand (6b),
on met en contact avec la surface (1) une première solution tampon contenant une molécule d'analyte (9) et, par une méthode de mesure analytique, on détecte les événements de liaison entre la molécule d'analyte (9) et le ligand (6a) et le deuxième ligand (6b),
lors d'une deuxième étape (II)
on met en contact une deuxième solution tampon avec la surface (1), de telle sorte que la liaison non covalente spécifique entre la première molécule (3) et la deuxième molécule (4) soit rompue, et que des molécules liées par liaison non covalente à la surface soient enlevées de la surface,
puis on répète au moins une fois les étapes (I) et (II).

2. Procédé selon la revendication 1, **caractérisé en ce que** le ligand (6a) est choisi dans le groupe consistant en les peptides, les protéines, les lipides, l'ADN, l'ARN, le PNA, le LNA, les oligosaccharides, ainsi que les composés à faible masse moléculaire, ayant une masse moléculaire ≤ 20 000 Da, de préférence ≤ 10 000 Da, d'une manière particulièrement préférée ≤ 3000 Da, en particulier ≤ 900 Da.

3. Procédé selon l'une des revendications précédentes, **caractérisé en ce que** le deuxième ligand (6b) est choisi dans le groupe consistant en les peptides, les protéines, les lipides, l'ADN, l'ARN, le PNA, le LNA, les oligosaccharides, ainsi que les composés à faible masse moléculaire, ayant une masse moléculaire ≤ 20 000 Da, de préférence ≤ 10 000 Da, d'une manière particulièrement préférée ≤ 3000 Da, en particulier ≤ 900 Da.

4. Procédé selon l'une des revendications précédentes, **caractérisé en ce que** le ligand (6a) et le deuxième ligand (6b) sont identiques ou différents.

5. Procédé selon l'une des revendications précédentes, **caractérisé en ce que** les événements de liaison sont détectés par spectroscopie par résonance plasmonique de surface (SPR), par une mesure d'interférences lumineuses (LIP) et/ou par des microbalances à cristaux de quartz (QCM).

6. Procédé selon l'une des revendications précédentes, **caractérisé en ce que** l'au moins une molécule d'analyte (9) est choisie dans le groupe consistant en les peptides, les protéines, les lipides, l'ADN, l'ARN, le PNA, le LNA, les oligosaccharides, ainsi que les composés à faible masse moléculaire ayant une masse moléculaire ≤ 20 000 Da, de préférence ≤ 10 000 Da, d'une manière particulièrement préférée ≤ 3000 Da, en particulier ≤ 900 Da.

7. Procédé selon l'une des revendications précédentes, **caractérisé en ce que** la surface bioactive (1) est, lors de l'étape (II), mise en contact avec la solution tampon, la solution tampon présentant un pH inférieur à 3.

8. Procédé selon l'une des revendications précédentes, **caractérisé en ce que** la surface bioactive (1) est, après rupture de la liaison non covalente spécifique, mise en contact avec un tampon de neutralisation, qui présente un pH compris dans la plage de 6 à 8.

9. Dispositif pour détecter des événements de liaison de molécules, dans lequel
au moins une première molécule (3) est immobilisée par une liaison covalente à une surface bioactive (1), la première molécule (3) étant choisie dans le groupe consistant en l'ADN, l'ARN, le LNA et le PNA simple brin, et
au moins une deuxième molécule (4) est liée par une liaison non covalente spécifique à l'au moins première molécule (3), l'au moins une deuxième molécule (4) étant choisie dans le groupe consistant en l'ADN, l'ARN, de LNA et le PNA simple brin, et la deuxième molécule (4) étant liée par liaison covalente chimique à un ligand (6a),
dans lequel au moins une troisième molécule (5) est, par l'intermédiaire d'au moins une liaison non covalente spécifique, liée à l'au moins une deuxième molécule (4), l'au moins une troisième molécule (5) étant choisie dans le groupe consistant en l'ADN, l'ARN, le LNA et le PNA simple brin, et l'au moins une troisième molécule (5) étant liée par liaison covalente à un deuxième ligand (6b),
la liaison non covalente spécifique de la première et de la deuxième molécule (3, 4) pouvant être rompue par mise en contact de la surface (1) avec une solution tampon.

10. Dispositif selon la revendication 9, **caractérisé en ce que** le ligand (6a) est choisi dans le groupe consistant en les peptides, les protéines, les lipides, l'ADN, l'ARN, le PNA, le LNA, les oligosaccharides, ainsi que les composés à faible masse moléculaire, ayant une masse moléculaire ≤ 20 000 Da, de préférence ≤ 10 000 Da, d'une manière particulièrement préférée ≤ 3000 Da, en particulier ≤ 900 Da.

11. Dispositif selon l'une des revendications 9 ou 10, **caractérisé en ce que** le deuxième ligand (6b) est choisi dans le groupe consistant en les peptides, les protéines, les lipides, l'ADN, l'ARN, le PNA, le LNA, les oligosaccharides, ainsi que les composés à faible masse moléculaire, ayant une masse moléculaire ≤ 20 000 Da, de préférence ≤ 10 000 Da, d'une manière particulièrement préférée ≤ 3000 Da, en particulier ≤ 900 Da.

12. Dispositif selon l'une des revendications 9 à 11, **caractérisé en ce que** le ligand (6a) et le deuxième ligand (6b) sont identiques ou différents.

13. Dispositif selon l'une des revendications 9 à 12, **caractérisé en ce que** la surface bioactive (1) convient à une mesure analytique d'événements de liaison, de préférence à une mesure analytique par une spectroscopie par résonance plasmonique de surface (SPR), à une mesure d'interférences lumineuses (LIM) et/ou à des microbalances à cristaux de quartz (QCM).

14. Dispositif selon l'une des revendications 9 à 13, **caractérisé en ce qu'**au moins une molécule d'analyte (9) est, de préférence par une liaison non covalente, liée à une surface bioactive (1), la molécule d'analyte étant de préférence choisie dans le groupe consistant en les peptides, les protéines, les lipides, l'ADN, l'ARN, le PNA, le LNA, les oligosaccharides, ainsi que les composés à faible masse moléculaire ayant une masse moléculaire ≤ 20 000 Da, de préférence ≤ 10 000 Da, d'une manière particulièrement préférée ≤ 3000 Da, en particulier ≤ 900 Da.

15. Dispositif selon l'une des revendications 9 à 14, **caractérisé en ce que** la liaison non covalente spécifique peut être rompue à l'aide d'une solution tampon qui présente un pH inférieur à 3.

16. Dispositif selon l'une des revendications 9 à 15, **caractérisé en ce que** la surface (1) est pour l'essentiel sèche.
